# EUROPEAN PATENT APPLICATION

(11) **EP 4 349 985 A1**
(43) Date of publication of application: **10.04.2024**
(21) Application number: 22815379.7
(22) Date of filing: 03.06.2022
(51) Int. Cl.: C12N 15/11, C12N 9/48, C07K 19/00, C12N 15/62, A61K 47/64, A61K 39/00, A61P 31/14

(54) **PEPTIDE TRANSLATED BY CIRCULAR RNA CIRC-ACE2 AND APPLICATION THEREOF**

(30) Priority: 04.06.2021 CN 202110624105; 07.07.2021 CN 202110765444
(71) Applicant: Guangzhou Institutes of Biomedicine and Health, Chinese Academy of Sciences, Guangzhou, Guangdong 510530 (CN)
(72) Inventor: LIU, Xingguo, Guangzhou, Guangdong 510530 (CN); LIU, Ming, Guangzhou, Guangdong 510530 (CN); ZHANG, Maolei, Guangzhou, Guangdong 510530 (CN); XING, Guangsuo, Guangzhou, Guangdong 510530 (CN); LIANG, Shan, Guangzhou, Guangdong 510530 (CN)
(74) Representative: DehnsGermany Partnerschaft von Patentanwälten
(86) International application number: PCT/CN2022/097024
(87) International publication number: WO 2022/253340

(57) **Abstract**

Provided are a polypeptide translated by a circular RNA Circ-ACE2 and an application thereof, the circular RNA molecule comprising at least one of the following sequences: 1) an RNA sequence as set forth in SEQ ID NO: 1; 2) an RNA sequence having at least 70% identity with 1), preferably an RNA sequence having at least 80% identity with 1), preferably an RNA sequences having at least 85% identity with 1), preferably an RNA sequence having at least 90% identity with 1), preferably an RNA sequence having at least 95% identity with 1), and more preferably, an RNA sequence having at least 99% identity with 1).

## Description

### PRIORITY INFORMATION

This application claims priority to and the benefit of Patent Application No. 202110624105.4, filed to the China National Intellectual Property Administration on June 04, 2021, and Patent Application No. 202110765444.4, filed to the China National Intellectual Property Administration on July 07, 2021, which are incorporated herein by reference in their entirety.

### TECHNICAL FIELD

The present disclosure relates to the field of biomedicine, in particular to a polypeptide translated by circular RNA Circ-ACE2 and a use thereof, and more particularly to an isolated circular RNA molecule, a polypeptide encoded by the isolated circular RNA molecule, a fusion protein, an isolated nucleic acid encoding the fusion protein, and use of the polypeptide encoded by the circular RNA molecule, the fusion protein, the isolated nucleic acid encoding the fusion protein in the preparation of a medicament or a kit, a method for inhibiting the infection of cells by COVID-19, a method for treating or preventing novel coronavirus pneumonia, and use of treating or preventing novel coronavirus pneumonia.

### BACKGROUND

A placenta is not only an essential temporary organ for the exchange of substances between the fetus and the mother during pregnancy, but also protects the fetus from infection by pathogenic microorganisms during fetal development. Placental cells may play an essential role in inhibiting the transmission of a virus from the pregnant mother to the fetus. Novel coronavirus (COVID-19) pandemics have a significant impact on global health. Some research groups reported that the mother-to-child vertical transmission of the COVID-19 virus is somewhat limited. The data showed that less than 5% of newborns are transmitted COVID-19 from their mothers, suggesting that the presence of unique antiviral substances in placental tissues can inhibit the virus transmission from the mother infected with COVID-19 to the fetus.

Polypeptide drugs have been widely used in clinical practices, and a typical representative is insulin polypeptide drugs. Metabolites of the polypeptide drugs are amino acids, which are essential elements for the human body. Polypeptide drugs are similar to protein drugs and thus have low toxicity and high safety. Compared with large protein and antibody drugs, polypeptide drugs have lower immunogenicity and better druggability, are easy to synthesize, and are suitable for injection and nasal nebulization administration.

Recent studies have found a large number of circular RNA molecules in a variety of organisms. Circular RNAs are widely found in eukaryotes and have essential biological functions in ontogeny and the occurrence and development of diseases. The epidemic caused by COVID-19 has been prevalent all over the world, and there are no specific therapeutic drugs with few side effects for diseases caused by the virus. There is a need to find and develop an ideal drug against COVID-19 from a new perspective.

### SUMMARY

The present application is based on the discovery and recognition of the following facts and issues by the inventors:

COVID-19 binds to ACE2 (angiotensin 2), thereby infecting cells. By in-depth analysis and experiments on the circAtlas comprehensive database of circular RNAs, the inventors found that the ACE2 gene was specifically cyclized and cleaved in human placental tissues, forming circular RNA Circ-ACE2. A polypeptide encoded by the circular RNA and a recombinant polypeptide obtained using the polypeptide have the ability to inhibit the infection of eukaryotic cells by COVID-19.

To this end, in a first aspect of the present disclosure, provided is an isolated circular RNA molecule. According to an embodiment of the present disclosure, the circular RNA molecule includes at least one of the following sequences: 1) an RNA sequence as set forth in SEQ ID NO: 1; 2) an RNA sequence having at least 70% identity with 1), preferably an RNA sequence having at least 80% identity with 1), preferably an RNA sequence having at least 85% identity with 1), preferably an RNA sequence having at least 90% identity with 1), preferably an RNA sequence having at least 95% identity with 1), and more preferably, an RNA sequence having at least 99% identity with 1).

According to an embodiment of the present disclosure, the specific sequence of the SEQ ID NO: 1 is:

CGCCCAACCCAAGUUCAAAGGCUGAUAAGAGAGAAAAUCUCAU
GAGGAGGUUUUAGUCUAGGGAAAGUCAUUCAGUGGAUGUGAUCUUGGCU
CACAGGGGACGAUGUCAAGCUCUUCCUGGCUCCUUCUCAGCCUUGUUGCU
GUAACUGCUGCUCAGUCCACCAUUGAGGAACAGGCCAAGACAUUUUUGG
ACAAGUUUAACCACGAAGCCGAAGACCUGUUCUAUCAAAGUUCACUUGCU
UCUUGGAAUUAUAACACCAAUAUUACUGAAGAGAAUGUCCAAAACAUG.

A polypeptide translated by the circular RNA sequence according to an embodiment of the present disclosure may significantly inhibit the ability of COVID-19 to infect cells.

According to an embodiment of the present disclosure, the above circular RNA molecule may further include at least one of the following additional technical features:

According to an embodiment of the present disclosure, the circular RNA molecule is formed by a separate cyclization of a second exon of an mRNA sequence encoding angiotensin-converting enzyme 2.

According to an embodiment of the present disclosure, the cyclization occurs in placental tissues.

According to an embodiment of the present disclosure, the circular RNA molecule is formed by linking a starting nucleotide and a terminal nucleotide of the nucleotide sequence as set forth in 1) or 2), the starting nucleotide being set as the first nucleotide of the circular RNA molecule.

According to an embodiment of the present disclosure, a coding region of the circular RNA includes nucleotides at position 104 to position 45 of the circular RNA molecule.

According to an embodiment of the present disclosure, a nucleic acid sequence of the coding region of the circular RNA includes at least one of the following sequences: 1) an RNA sequence as set forth in SEQ ID NO: 2; 2) an RNA sequence having at least 70% identity with 1), preferably an RNA sequence having at least 80% identity with 1), preferably an RNA sequence having at least 85% identity with 1), preferably an RNA sequence having at least 90% identity with 1), preferably an RNA sequence having at least 95% identity with 1), and more preferably, an RNA sequence having at least 99% identity with 1).

According to an embodiment of the present disclosure, the specific sequence of the SEQ ID NO: 2 is:

AUGUCAAGCUCUUCCUGGCUCCUUCUCAGCCUUGUUGCUGUAA
CUGCUGCUCAGUCCACCAUUGAGGAACAGGCCAAGACAUUUUUGGACAAG
UUUAACCACGAAGCCGAAGACCUGUUCUAUCAAAGUUCACUUGCUUCUUG
GAAUUAUAACACCAAUAUUACUGAAGAGAAUGUCCAAAACAUGCGCCCA
ACCCAAGUUCAAAGGCUGAUAAGAGAGAAAAUCUCAUGA.

A polypeptide translated by the nucleic acid sequence of the coding region of the circular RNA according to an embodiment of the present disclosure may significantly inhibit the ability of COVID-19 to infect cells.

In a second aspect of the present disclosure, provided is a polypeptide. According to an embodiment of the present disclosure, an amino acid sequence of the polypeptide includes at least one of the following sequences: 1) an amino acid sequence as set forth in SEQ ID NO: 3; 2) an amino acid sequence having at least 70% identity with 1), preferably an amino acid sequence having at least 80% identity with 1), preferably an amino acid sequence having at least 85% identity with 1), preferably an amino acid sequence having at least 90% identity with 1), preferably an amino acid sequence having at least 95% identity with 1), and more preferably, an amino acid sequence having at least 99% identity with 1). According to an embodiment of the present disclosure, the polypeptide may significantly inhibit the ability of COVID-19 to infect cells.

According to an embodiment of the present disclosure, the specific sequence of the SEQ ID NO: 3 is:

MSSSSWLLLSLVAVTAAQSTIEEQAKTFLDKFNHEAEDLFYQSSLASW
NYNTNITEENVQNMRPTQVQRLIREKIS.

According to an embodiment of the present disclosure, the above polypeptide may further include at least one of the following additional technical features:

According to an embodiment of the present disclosure, the polypeptide includes a signal peptide sequence.

According to an embodiment of the present disclosure, an amino acid sequence of the signal peptide is MSSSSWLLLSLVAVTAA.

According to an embodiment of the present disclosure, the polypeptide is present in placental tissues.

In a third aspect of the present disclosure, provided is a fusion protein. According to an embodiment of the present disclosure, the fusion protein includes the polypeptide according to the second aspect and an Fc, wherein the C-terminal of the peptide is linked to the N-terminal of the Fc. According to an embodiment of the present disclosure, the fusion protein may significantly inhibit the ability of COVID-19 to infect cells.

According to an embodiment of the present disclosure, the fusion protein may further include at least one of the following additional technical features:

According to an embodiment of the present disclosure, the fusion protein includes at least one of the following sequences: 1) an amino acid sequence as set forth in SEQ ID NO: 4; 2) an amino acid sequence having at least 70% identity with 1), preferably an amino acid sequence having at least 80% identity with 1), preferably an amino acid sequence having at least 85% identity with 1), preferably an amino acid sequence having at least 90% identity with 1), preferably an amino acid sequence having at least 95% identity with 1), and more preferably, an amino acid sequence having at least 99% identity with 1).

According to an embodiment of the present disclosure, the specific sequence of the SEQ ID NO: 4 is:

MSSSSWLLLSLVAVTAAQSTIEEQAKTFLDKFNHEAEDLFYQSSLASW
NYNTNITEENVQNMRPTQVQRLIREKISLVPRGSGGGDPEPKSCDKTHTCPPCP
APELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNY
KTTPPVLDSDGSFFLYSKLTVDKSRWQQGNVFSCSVMHEALHNHYTQKSLSLSP
GK.

In a fourth aspect of the present disclosure, provided is an isolated nucleic acid. According to an embodiment of the present disclosure, the isolated nucleic acid encodes the polypeptide according to the second aspect or the fusion protein according to the third aspect. The isolated nucleic acid according to an embodiment of the present disclosure may be expressed under a suitable condition to obtain a large amount of the polypeptide or the fusion protein. Both of the polypeptide translated by the isolated nucleic acid and the fusion protein may significantly inhibit the ability of COVID-19 to infect cells.

According to an embodiment of the present disclosure, the isolated nucleic acid may further include at least one of the following additional technical features:

According to an embodiment of the present disclosure, the nucleic acid encoding the fusion protein includes at least one of the following sequences: 1) a nucleotide sequence as set forth in SEQ ID NO: 5; 2) a nucleotide sequence having at least 70% identity with 1), preferably a nucleotide sequence having at least 80% identity with 1), preferably a nucleotide sequence having at least 85% identity with 1), preferably a nucleotide sequence having at least 90% identity with 1), preferably a nucleotide sequence having at least 95% identity with 1), and more preferably, a nucleotide sequence having at least 99% identity with 1).

According to an embodiment of the present disclosure, the specific sequence of the SEQ ID NO: 5 is:

ATGTCAAGCTCTTCCTGGCTCCTTCTCAGCCTTGTTGCTGTAACTG
CTGCTCAGTCCACCATTGAGGAACAGGCCAAGACATTTTTGGACAAGTTTAA
CCACGAAGCCGAAGACCTGTTCTATCAAAGTTCACTTGCTTCTTGGAATTATA
ACACCAATATTACTGAAGAGAATGTCCAAAACATGCGCCCAACCCAAGTTCA
AAGGCTGATAAGAGAGAAAATCTCACTGGTGCCCAGAGGCTCCGGCGG
CGGCGATCCTGAGCCCAAATCTTGTGACAAAACTCACACATGCCCACCGTGC
CCAGCACCTGAACTCCTGGGGGGACCGTCAGTCTTCCTCTTCCCCCCAAAAC
CCAAGGACACCCTCATGATCTCCCGGACCCCTGAGGTCACATGCGTGGTGGT
GGACGTGAGCCACGAAGACCCTGAGGTCAAGTTCAACTGGTACGTGGACGG
CGTGGAGGTGCATAATGCCAAGACAAAGCCGCGGGAGGAGCAGTACAACAG
CACGTACCGTGTGGTCAGCGTCCTCACCGTCCTGCACCAGGACTGGCTGAAT
GGCAAGGAGTACAAGTGCAAGGTCTCCAACAAAGCCCTCCCAGCCCCCATC
GAGAAAACCATCTCCAAAGCCAAAGGGCAGCCCCGAGAACCACAGGTGTAC
ACCCTGCCCCCATCCCGGGATGAGCTGACCAAGAACCAGGTCAGCCTGACCT
GCCTGGTCAAAGGCTTCTATCCCAGCGACATCGCCGTGGAGTGGGAGAGCAA
TGGGCAGCCGGAGAACAACTACAAGACCACGCCTCCCGTGCTGGACTCCGA
CGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACT
ACACGCAGAAGAGCCTCTCCCTGTCTCCGGGTAAATGA.

In a fifth aspect of the present disclosure, provided is an expression vector. According to an embodiment of the present disclosure, the expression vector includes the RNA molecule according to the first aspect or the isolated nucleic acid according to the fourth aspect. A polypeptide translated by the expression vector according to an embodiment of the present disclosure may significantly inhibit the ability of COVID-19 to infect cells.

In a sixth aspect of the present disclosure, provided is a recombinant cell. According to an embodiment of the present disclosure, the recombinant cell carries the RNA molecule according to the first aspect, the isolated nucleic acid according to the fourth aspect, or the expression vector according to the fifth aspect. The recombinant cell has a stronger ability to resist the infection by COVID-19.

In a seventh aspect of the present disclosure, provided is a pharmaceutical composition. According to an embodiment of the present disclosure, the pharmaceutical composition includes the RNA molecule according to the first aspect, the polypeptide according to the second aspect, the fusion protein according to the third aspect, the isolated nucleic acid according to the fourth aspect, the expression vector according to the fifth aspect, or the recombinant cell according to the sixth aspect. According to an embodiment of the present disclosure, the pharmaceutical composition may be used to resist the infection of cells by COVID-19.

According to an embodiment of the present disclosure, the pharmaceutical composition may further include at least one of the following additional technical features:

According to an embodiment of the present disclosure, the pharmaceutical composition is in at least one form selected from a group consisted of a solution, a powder, a microsphere, and a microcapsule. Further, the pharmaceutical composition according to an embodiment of the present disclosure is conveniently administered and suitable for maintaining the pharmaceutical effect.

According to an embodiment of the present disclosure, a dosage of the pharmaceutical composition is not particularly limited and, in practice, may be flexibly selected based on the health condition of a subject to be administered.

In an eighth aspect of the present disclosure, provided is the use of the RNA molecule according to the first aspect, the polypeptide according to the second aspect, the fusion protein according to the third aspect, the isolated nucleic acid according to the fourth aspect, the expression vector according to the fifth aspect, the recombinant cell according to the sixth aspect, or the pharmaceutical composition according to the seventh aspect in the preparation of a medicament or a kit for inhibiting the infection of cells by COVID-19. According to an embodiment of the present disclosure, the medicament or the kit may significantly inhibit the ability of COVID-19 to infect cells and may be used for clinical diagnosis or scientific research.

In a ninth aspect of the present disclosure, provided is a method for inhibiting the infection of cells by COVID-19, including: contacting COVID-19 or cells to be infected with the RNA molecule according to the first aspect, the polypeptide according to the second aspect, the fusion protein according to the third aspect, the isolated nucleic acid according to the fourth aspect, the expression vector according to the fifth aspect, the recombinant cell according to the sixth aspect, or the pharmaceutical composition according to the seventh aspect. According to an embodiment of the present disclosure, the method may significantly inhibit the infection of cells by COVID-19.

According to an embodiment of the present disclosure, the above method may further include at least one of the following additional technical features:

According to an embodiment of the present disclosure, the contacting is *in vivo* or *ex vivo.* For example, the *in vivo* contacting may be achieved by at least one approach of direct microinjection, oral administration, nasal administration, transdermal administration, intravenous injection, respiratory inhalation, and rectal administration, that is, by introducing the RNA molecule according to the first aspect, the polypeptide according to the second aspect, the fusion protein according to the third aspect, the isolated nucleic acid according to the fourth aspect, the expression vector according to the fifth aspect, the recombinant cell according to the sixth aspect, or the pharmaceutical composition according to the seventh aspect into a subject to contact with cells to be infected, such as lung epithelial cells, by the approaches described above. For example, the *ex vivo* contacting means that the RNA molecule according to the first aspect, the polypeptide according to the second aspect, the fusion protein according to the third aspect, the isolated nucleic acid according to the fourth aspect, the expression vector according to the fifth aspect, the recombinant cell according to the sixth aspect, or the pharmaceutical composition according to the seventh aspect is directly mixed, incubated or contacted with COVID-19 or isolated cells to be infected *in vitro.*

According to a particular embodiment of the present disclosure, the contacting is carried out by the following steps: 1) performing a first mixing treatment of COVID-19 with the RNA molecule according to the first aspect, the polypeptide according to the second aspect, the fusion protein according to the third aspect, the isolated nucleic acid according to the fourth aspect, the expression vector according to the fifth aspect, the recombinant cell according to the sixth aspect, or the pharmaceutical composition according to the seventh aspect; 2) performing a second mixing treatment of the mixture obtained in step 1) with the cells to be infected to inhibit the infection of the cells by COVID-19.

According to an embodiment of the present disclosure, the final concentration of the polypeptide or the fusion protein in a mixed system is 10 µg/mL or 160 µg/mL. According to an embodiment of the present disclosure, the polypeptide at a final concentration of 10 µg/mL in the mixed system may inhibit the ability of COVID-19 to infect cells by 91.61%, and the polypeptide at a final concentration of 160 µg/mL may inhibit the ability of COVID-19 to infect cells by 95.50%. The fusion protein (recombinant polypeptide) at a final concentration of 10 µg/mL in the mixed system may inhibit the ability of COVID-19 to infect cells by 52.11%, and the fusion protein at a final concentration of 160 µg/mL may inhibit the ability of COVID-19 to infect cells by 92.18%. The administration of the polypeptide or the fusion protein at the final concentration in the mixed system according to the embodiment of the present disclosure is suitable for maintaining the pharmaceutical effect.

In a tenth aspect of the present disclosure, provided is a method for treating or preventing novel coronavirus pneumonia. According to an embodiment of the present disclosure, the pharmaceutically acceptable RNA molecule, the polypeptide, the fusion protein, or the isolated nucleic acid as described above is administered to a patient. The method according to an embodiment of the present disclosure may effectively treat or prevent novel coronavirus pneumonia.

In an eleventh aspect of the present disclosure, provided is the use of the RNA molecule, the polypeptide, the fusion protein, or the isolated nucleic acid as described above in the treatment or prevention of novel coronavirus pneumonia.

Additional aspects and advantages of the present disclosure will be set forth in part in the following description and, in part, will become apparent from the description or may be learned by the practice of the present disclosure.

### BRIEF DESCRIPTION OF DRAWINGS

The above and/or additional aspects and advantages of the present disclosure will become apparent and be readily appreciated from the following description of embodiments with reference to the accompanying drawings, in which:
FIG. 1 is a distribution diagram showing an expression level of an ACE2 mRNA molecule in various human tissues according to an embodiment of the present disclosure;
FIG. 2 is a distribution diagram showing an expression level of circular RNA circ-ACE2 in various human tissues according to an embodiment of the present disclosure;
FIG. 3 is a pattern diagram showing the formation of the circular RNA Circ-ACE2 according to an embodiment of the present disclosure, wherein the ACE2 RNA is cleaved and cyclized at a second exon (Exon2) to form circular the RNA Circ-ACE2;
FIG. 4 is a diagram showing the results of sequencing PCR products of RNA Circ-ACE2 to verify a cyclized sequence of circular RNA Circ-ACE2 according to an embodiment of the present disclosure, wherein the direction of the PCR result diagram from left to right is from the 3' end to the 5' end of the PCR product sequence;
FIG. 5 is a diagram showing the analysis of a coding reading frame spanning a junction of circular RNA Circ-ACE2 according to an embodiment of the present disclosure, wherein the coding sequence of the circular RNA molecule is a nucleotide sequence from nucleotide at position 104 to nucleotide at position 45 of the circular RNA molecule sequence spanning the cyclized junction, the specific sequence being underlined in the figure;
FIG. 6 is a diagram showing the alignment and analysis of an amino acid sequence of CircACE2-76aa polypeptide and that of a protein encoded by ACE2 according to an embodiment of the present disclosure, wherein, aligned with the amino acid sequence of the ACE2 protein, CircACE2-76aa contains N-terminal 62 amino acids of ACE2 and a C-terminal carrying a specific 14 amino acids (RPTQVQRLIREKIS);
FIG. 7 is a diagram showing the results of mass spectrometric identification of a polypeptide translated by a circular RNA Circ-ACE2 molecule according to an embodiment of the present invention;
FIG. 8 is an online program analysis chart of an amino acid sequence of CircACE2-76aa according to an embodiment of the present disclosure;
FIG. 9 is cellular localization pictures of CircACE2-76aa under a fluorescence microscope according to an embodiment of the present disclosure;
FIG. 10 is a diagram showing the results of detecting the fold difference in an expression level of an EGFP gene carried by a novel coronavirus pseudovirus in an experiment for infecting cells with the pseudovirus using a chemically synthesized polypeptide CircACE2-76aa according to an embodiment of the present disclosure; and
FIG. 11 is a diagram showing the results of detecting the fold difference in an expression level of an EGFP gene carried by a novel coronavirus pseudovirus in an experiment for infecting cells with the pseudovirus using recombinant protein CircACE2-76aa-FC according to an embodiment of the present disclosure.

### DESCRIPTION OF EMBODIMENTS

Embodiments of the present disclosure will be described in detail below, examples of which are illustrated in the accompanying drawings. The embodiments described below with reference to the accompanying drawings are illustrative and are merely intended to explain the present disclosure rather than to limit the present disclosure.

### Explanation of terms

As used herein, both of the terms "novel coronavirus" and "COVID-19" refer to a pathogen causing novel coronavirus pneumonia.

With regard toidentitiy, in the present disclosure, to compare two or more nucleotide sequences, the percentage of "sequence identity" between a first sequence and a second sequence may be calculated by [dividing the number of nucleotides in the first sequence that are identical to nucleotides at the corresponding position]. Nucleotides in the second sequence] minus [total number of nucleotides in the first sequence] and then multiplied by [100%], wherein the deletion, insertion, substitution, or addition of each nucleotide in the second nucleotide sequence, relative to the first nucleotide sequence, is considered to be a difference in a single nucleotide (position).

Alternatively, the degree of sequence identity between two or more nucleotide sequences may be calculated using a standard setting using known computer algorithms for sequence alignment, such as NCBI Blast v2.0.

Some other techniques, computer algorithms, and settings for determining the degree of sequence identity are, for example, described in WO 04/037999, EP 0 967 284, EP 1 085 089, WO 00/55318, WO 00/78972, WO 98/49185, and GB 2357768-A.

With regard to identity, in the present disclosure, to compare two or more amino acid sequences, the percentage of "sequence identity" between a first amino acid sequence and a second amino acid sequence may be determined by [dividing the number of amino acid residues in the first amino acid sequence that are identical to [amino acid residues at the corresponding position in the second amino acid sequence by the total number of nucleotides in the first amino acid sequence] and then multiplying by 100%, wherein each deletion, insertion, substitution or addition of a "residue" of amino acid residues in the second amino acid sequence relative to the first amino acid sequence is considered a difference in a single amino acid residue (position), i.e., an "amino acid difference" as defined herein.

Alternatively, the degree of sequence identity between two amino acid sequences may be calculated again using a standard setting using known computer algorithms, such as the above algorithms for determining the degree of sequence identity of nucleotide sequences.

In general, to determine the percentage of "sequence identity" between two amino acid sequences based on the calculation method outlined above, the amino acid sequence having the largest number of amino acid residues is taken as the "first" amino acid sequence, and the other amino acid sequence is taken as the "second" amino acid sequence.

Likewise, when determining the degree of sequence identity between two amino acid sequences, the skilled person may consider so-called "conservative" amino acid substitutions, which may generally be described as amino acid substitutions in which an amino acid residue is replaced byanother amino acid residue with a similar chemical structure, taking little or no effect on the function, activity, or other biological properties of the polypeptide. Such conservative amino acid substitutions are well known in the art, for example, from WO 04/037999, GB-A-2357768, WO 98/49185, WO 00/46383, and WO 01/09300 and WO 01/09300. The type and/or combination of such substitutions may be selected and/or (preferably) based on the relevant teachings from WO 04/037999 and WO 98/49185 and other references cited therein.

By in-depth analysis on the circAtlas comprehensive database of circular RNAs, the inventors found that the ACE2 gene specifically forms a circular RNA molecule in placental tissue. The circular RNA molecule is formed by a separate cyclization of a second exon of ACE2 RNA, named Circ-ACE2. The sequence analysis and experimental verification of the circular RNA Circ-ACE2 suggest that CircACE2 can translate a polypeptide that includes a secreted signal peptide. The polypeptide is a novel polypeptide molecule, named CircACE2-76aa, with a length of 76 amino acids. In the present disclosure, the recombinant polypeptide CircACE2-76aa-FC is obtained by chemically synthesizing the polypeptide CircACE2-76aa and expressing an FC fragment of human immunoglobulin G fused with the CircACE2-76aa in eukaryotic cells, and the antiviral function of CircACE2-76aa is verified using a strategy that human hACE2-293T cells are infected with COVID-19 pseudovirus *in vitro.* The results show that the chemically synthesized polypeptide CircACE2-76aa and recombinant polypeptide CircACE2-76aa-FC can significantly inhibit the ability of COVID-19 pseudovirus to infect cells. The polypeptide CircACE2-76aa and recombinant polypeptide CircACE2-76aa-FC involved in the present disclosure may be candidate molecules for inhibiting COVID-19 virus infection.

Examples will be described in detail below. The experimental methods applied in the following examples are conventional methods, unless otherwise specified. The materials, reagents, and the like used in the following examples are all commercially available, unless otherwise specified.

### Example 1. Recognition and identification of circular RNA Circ-ACE2 in isolated placental tissues

Human placental tissues in this example were derived from discarded placentas of pregnant women after natural delivery in the department of clinical gynaecology and obstetrics.

### 1. Recognition of the circular RNA Circ-ACE2 in isolated placental tissues

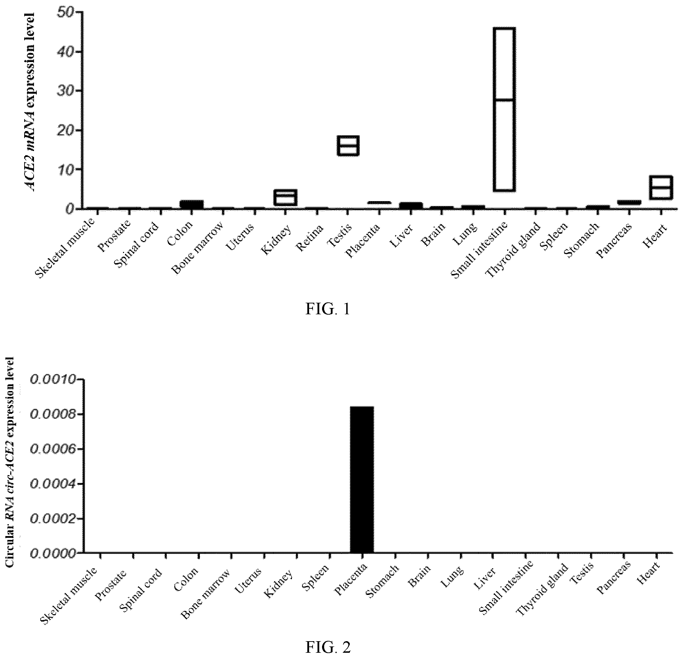

ACE2 mRNA was widely expressed in many human tissues, and its expression distribution in various human tissues was shown in FIG. 1. By in-depth analysis on the circAtlas comprehensive database of circular RNAs, the inventors found that the ACE2 gene was cyclized and cleaved specifically in isolated placental tissues, forming a circular RNA Circ-ACE2, as shown in FIG. 2.

### 2. Sequencing identification of the circular RNA Circ-ACE2 in isolated placenta tissues

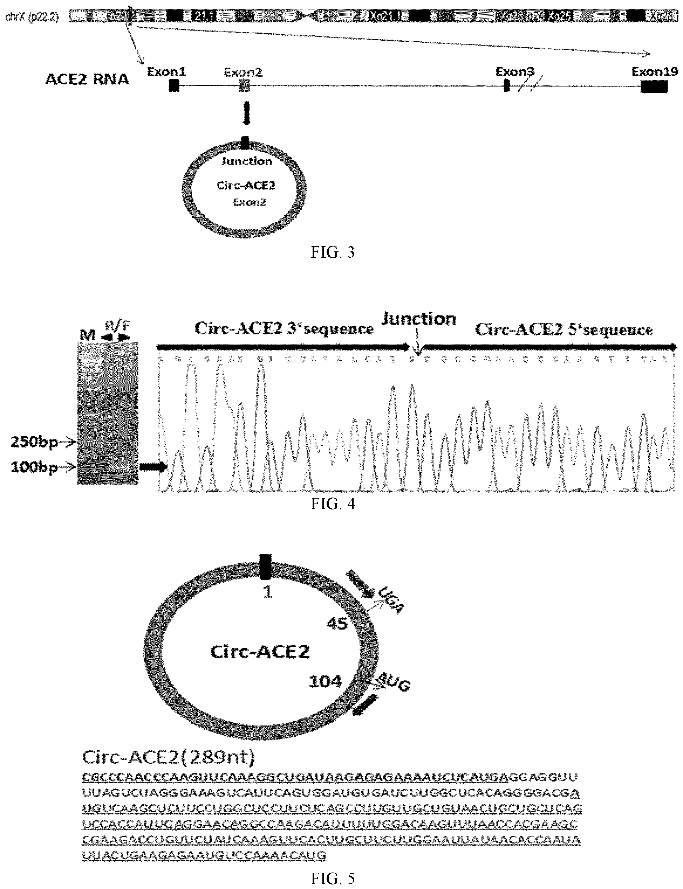

PCR detection primers were designed for specifically detecting the circular RNA Circ-ACE2, wherein an upstream primer sequence was 5' GAAGCCGAAGACCTGTTCTA 3', a downstream primer sequence was 5' TCTTATCAGCCTTTGAACTTGG 3', and the amplified fragment was 114bp in size. The total RNA of isolated placental tissue samples was extracted using a Trizol method, and a reverse transcription kit (Vazyme Corporation) was used to reverse-transcribe the RNA into cDNA using random primers. The reaction system and conditions for PCR amplification were as follows: a 50 µL of PCR total system, including 25 µL of 2x PCR MIX (Vazyme Corporation), 2 µL each of upstream and downstream primers (10 mM), 1 µL of cDNA template, and made up to 50 µL with sterile water. The reaction conditions were: cDNA pre-denaturation at 95°C for 3 min; first step amplification: denaturation at 95°C for 30 s, annealing at 60°C for 30 s, extension at 72°C for 30 s, amplification for 35 cycles with the final extension at 72°C for 3 min, followed by storage at 16°C. PCR products were separated on a 1.5% agarose gel, cut, and purified for Sanger DNA sequencing. As can be seen in FIG. 3, the circular RNA Circ-ACE2 was a circular RNA molecule formed by the separate cyclization and cleavage of a second exon of ACE2 mRNA. As shown in FIG. 4, the inventors identified an exact cyclized junction of the circular RNA Circ-ACE2 by Sanger DNA sequencing for the PCR products. The Circ-ACE2 RNA sequence had a length of 289nt and the specific nucleic acid sequence as shown in SEQ ID NO: 1.

### 3. Coding potential analysis for the RNA Circ-ACE2

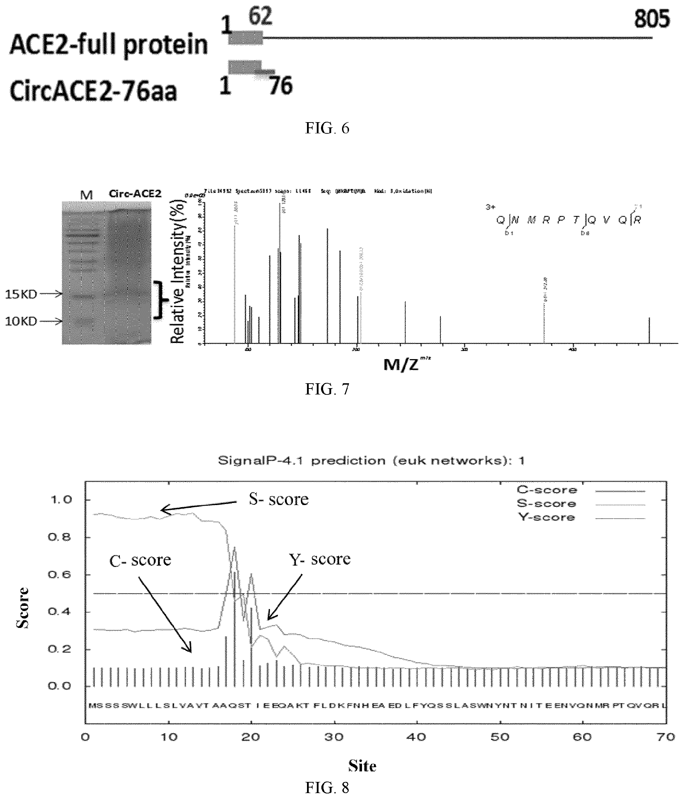

Coding potential analysis was performed using the ORF finder online program based on the nucleic acid sequence of the RNA Circ-ACE2. It was found that the circular RNA Circ-ACE2 contained a reading frame spanning a circular RNA junction, as shown in FIG. 5. A coding region of a polypeptide translated by the circular RNA Circ-ACE2 consisted of 231 bases, and the base sequence was shown in SEQ ID NO: 2. The polypeptide translated by the circular RNA Circ-ACE2 had a length of 76 amino acids, named CircACE2-76aa in the present disclosure, with the amino acid sequence shown in SEQ ID NO: 3. The amino acid sequence of the CircACE2-76aa was aligned with that of a protein encoded by the RNA ACE2. The results showed that the CircACE2-76aa contained N-terminal 62 amino acids of ACE2 and a C-terminal carrying a specific 14 amino acids (RPTQVQRLIREKIS), as shown in FIG. 6.

### Example 2. Identification of the polypeptide CircACE2-76aa translated by the circular RNA Circ-ACE2

### 1. Design and construction of a circular RNA Circ-ACE2 expression plasmid

A target sequence was obtained by a whole-gene chemical synthesis method based on the sequence information of the circular RNA Circ-ACE2 and then constructed into a circular RNA expression vector pCD5-ciR (Geneseed) by restriction enzymes EcoRI and BamHI.

### 2. Construction of a red fluorescent protein fusion expression plasmid CircACE2-76aa-mcherry

According to the circular RNA expression design method of a circular RNA expression vector CircRNA Mini Vector (Addgene ID: # 60648), a circular RNA expression vector CircACE2-76aa-mcherry that fused the CircACE2-76aa with a red fluorescent protein mcherry was designed. The start codon ATG and the stop codon of the mcherry gene fragment itself were removed, and then the resulting fragment was inserted into the front end of the stop codon of the CircACE2-76aa. The cyclization-mediated sequence of the CircRNA Mini Vector was added at both ends of the sequence, and a finally designed expression cassette was shown in SEQ ID NO: 7. After a CircACE2-76aa-mcherry circular RNA fusion expression cassette was designed, the expression cassette was obtained by a whole-gene chemical synthesis method, and then was constructed into a pcDNA3.1(+) expression vector by using EcoRI and BamHI restriction enzyme sites.

### 3. Transfection of hACE2-293T cells with the CircACE2-76aa-mcherry

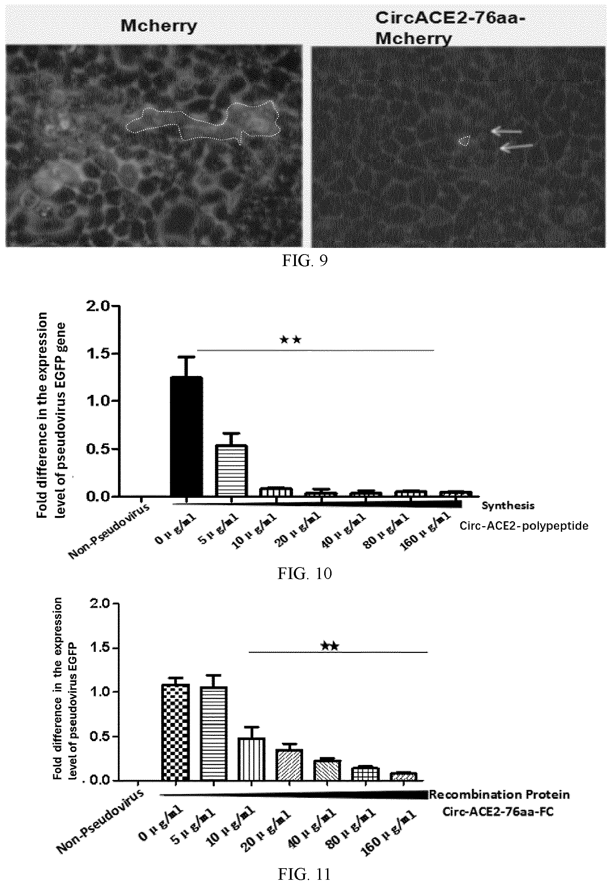

The circular RNA Circ-ACE2 expression plasmid was transfected into 293T cells, and then circular RNA Circ-ACE2 expression products were subjected to SDS-PAGE protein electrophoresis, Coomassie brilliant blue staining and cutting gel for mass spectrometry identification. As shown in Figure 7, a peptide segment spanning a cyclized junction translated by the circular RNA Circ-ACE2 was identified in mass spectrometry. The sequence of the peptide segment obtained by mass spectrometry was QNMRPTAVQR, and the sequence showed a terminal-specific amino acid sequence translated by the non-circular RNA circ-ACE2. The analysis of the amino acid sequence of the CircACE2-76aa polypeptide using the signalP-4.1 online program showed that the circACE2-76aa did not include a transmembrane amino acid sequence and included a typical secreted signal peptide sequence, as shown in FIG. 8. The secreted signal peptide sequence of the CircACE2-76aa consisted of 17 amino acids at the N-terminal, with a sequence of MSSSSWLLLSLVAVTAA. Localization of the CircACE2-76aa polypeptide was observed by fluorescence microscopy using the fused red fluorescent protein mcherry, and the CircACE2-76aa polypeptide was found to be secreted extracellularly, as shown in Fig. 9.

### Example 3. A chemical synthesis method of peptide CircACE2-76aa

In this example, Shanghai Botai Biological Company was entrusted to carry out the chemical synthesis of the polypeptide using a solid phase synthesis method. A Fmoc/PyBOP method was used to synthesize the peptide chain. The Fmoc protecting group was removed with 30% piperidine in DMF. The peptide chain was cleaved from a resin with a peptide cleavage reagent (trifluoroacetic acid/crystalline phenol/water/ethanedithiol/methyl ethyl sulfide/= 81.5/5/5/5/2.5/1). The obtained peptide was purified and detected using a C18 reverse phase column under the conditions that phase A was 95% water (methanol ratio), phase B was 95% methanol (methanol ratio), and then 0.1% TFA was added to each phase. The following conventional conditions were used: equilibrating the column with phase A for 15 minutes before loading samples and then loading the samples with a 25-minute gradient from phase A to phase B. Detecting wavelength: 220 nm, flow rate: 1 mL/min. The column was first equilibrated with solution A. After loading, the samples were gradient-eluted from solution A to solution B for 25 min, and a target peptide was collected. The synthesized polypeptide was identified by mass spectrometry.

### Example 4. Construction of a circACE2-76aa-FC polypeptide recombinant expression plasmid and expression and purification in eukaryotic cells

### 1. Construction of the polypeptide recombinant expression plasmid

A recombinant expression cassette was designed based on a nucleic acid sequence encoding the circACE2-76aa and a human immunoglobulin IGg sequence, and the nucleic acid sequence thereof was shown in SEQ ID NO: 5. A circACE2-76aa-FC expression cassette was obtained using a strategy of segmented PCR amplification and overlapping PCR splicing amplification, and then ligated into a pcDNA3.1(+) vector by EcoRI and BamHI restriction enzyme sites.

### 2. Transfection of the expression plasmid into eukaryotic cells

The cells were cultured to a density of 2.5-3.0 million/mL, and then 500 mL of the cells were cultured in a 2 L culture flask at a temperature of 37°C, a rotation speed of 120 rpm, and 8% CO₂. The plasmid was transfected based on 0.5 mg of the plasmid per 500 mL of the cells, with the amount of PEI being 2.7 times the amount of the plasmid. 10 mL of fresh culture medium was first prepared (taking 500 mL as an example), and 50 µg of the plasmid was added and mixed well. The corresponding amount of PEI was slowly added and incubated for 5-10 min (it could be observed that the culture medium changed from an original transparent state to a slightly turbid state). After the end of incubation, the mixture was added to the cells which then were allowed to express at 33°C, 8% CO₂, and a rotation speed of 120 rpm. A cell supernatant was collected to stop the expression after 6 days of expression.

### 3. Purification of the circACE2-76aa-FC recombinant polypeptide

5 mL of neutralization solution (1 M tris-HCl, pH 8.0) was added to each collection tube. An AKTA prue column (AKTA prue column containing protein A (Cytiva Corporation, Cat: 17040301)) was equilibrated with Binding buffer (20 mM phosphate), and 500 mL of the cell supernatant was loaded to the purification column set to a volume of 1500 mL, washed 80 mL of impure protein with Binding buffer (20 mM phosphate), eluted 100 mL with Elution buffer (0.1 M citric acid, pH 3.0), and collected 5 mL in each collection tube. Collection tubes at a peak position were taken to run a gel, and a collection tube with the protein was determined. The protein was concentrated and finally replaced into PBS.

### Example 5. Experimental testing of the infection of cells by COVID-19 novel coronavirus pseudovirus for the CircACE2-76aa polypeptide and the recombinant CircACE2-76aa-FC

A commercial COVID-19 S protein recombinant pseudovirus and a 293T cell line hACE2-293T stably overexpressing a human ACE2 full-length gene were used to simulate a process of the virus invasion into cells. The chemically synthesized CircACE2-76aa polypeptide (SEQ ID NO: 6) and recombinant polypeptide CircACE2-76aa-FC (SEQ ID NO: 4) obtained by eukaryotic expression in 293T were mixed and incubated with the novel coronavirus pseudovirus respectively, and then the hACE2-293T cells were infected with the mixture. The specific operations were as follows. The hACE2-293T cells were plated in a 96-well plate at a density of 1×10⁴ cells/well. After 12 hours, the cells were adherent and used to perform a pseudovirus COVID-19 infection experiment. 5 µL of the pseudovirus (about 10,000 virus particles) and different concentrations of the polypeptides were added to the cells per well. The pseudovirus and the polypeptides were previously mixed in 100 µL of DMEM complete medium *in vitro* according to a ratio, and the mixture was placed at room temperature for 30 min. The cells were added with polybrene at a final concentration of 8 µg/mL before being infected with the pseudovirus.

The genomic DNA of the infected cells was extracted and used to perform a QPCR fluorescence quantitative assay based on an EGFP gene sequence carried by the novel coronavirus pseudovirus to quantitatively detect the effects of the chemically synthesized CircACE2-76aa polypeptide and the recombinant CircACE2-76aa-FC on inhibiting the infection of cells by the novel coronavirus pseudovirus COVID-19. The specific operations were as follows. The hACE2-293T cells were infected with the pseudovirus for 15 h. The cell culture medium was removed, and 100 µL of cell genome DNA extraction and lysis buffer was directly added to each well. The cells were mixed and lysed in a 96-well plate and transferred to a 1.5 mL centrifuge tube. The genome DNA was then extracted and dissolved in sterile water. 1 µL of the genome DNA was taken to perform the QPCR fluorescence quantitative assay to detect the integration of the EGFP gene carried by the pseudovirus into the cell genome. The GAPDH gene was used as an internal reference to evaluate the ability of the novel coronavirus pseudovirus to infect the hACE2-293T cells.

QPCR assay: The reaction system for the QPCR assay was prepared according to the instructions of a QPCR kit (Vazyme Company). The reaction system for QPCR was 20 µL, including 10 µL of 2x SYBR Green PCR Master Mix, 0.4 µL each of upstream and downstream primers (10 µM), 1 µL of template DNA, and made up to 20 µL with sterile deionized water. The reaction conditions for fluorescent quantitative PCR were: cDNA pre-denaturation at 95°C for 5 min; first step amplification: denaturation at 95°C for 10 s, annealing at 60°C for 35 s (this step collects fluorescence signals); 40 cycles, followed by melting curve analysis: fluorescence signals collection at 60-95°C. The primer sequences for the QPCR assay were: EGFP-upstream primer: 5' TTCAAGGAGGACGGCAACAT 3', EGFP-downstream primer: 5' TGGCGGATCTTGAAGTTCAC 3', and the amplified product was 119bp in size.

As can be seen from FIG. 10, the chemically synthesized polypeptide CircACE2-76aa at a concentration of 10 µg/mL could inhibit the ability of the novel coronavirus pseudovirus to infect cells by 91.61%, and the chemically synthesized polypeptide CircACE2-76aa at a concentration of 160 µg/mL could inhibit the ability of the novel coronavirus pseudovirus to infect cells by 95.50%. FIG. 11 shows that the recombinant CircACE2-76aa-FC at a concentration of 10 µg/mL could inhibit the ability of the novel coronavirus pseudovirus to infect cells by 52.11%, and the recombinant CircACE2-76aa-FC at a concentration of 160 µg/mL could inhibit the ability of the novel coronavirus pseudovirus to infect cells by 92.18%, as shown in FIG. 11.

**Table 1:**

| Name | Sequence |
|---|---|
| Circular RNA Circ-ACE2 sequence | CGCCCAACCCAAGUUCAAAGGCUGAUAAGAGAGAAAAU CUCAUGAGGAGGUUUUAGUCUAGGGAAAGUCAUUCAG UGGAUGUGAUCUUGGCUCACAGGGGACGAUGUCAAGC UCUUCCUGGCUCCUUCUCAGCCUUGUUGCUGUAACUGCU GCUCAGUCCACCAUUGAGGAACAGGCCAAGACAUUUUUGG ACAAGUUUAACCACGAAGCCGAAGACCUGUUCUAUCAAA GUUCACUUGCUUCUUGGAAUUAUAACACCAAUAUUACUGA |
| | AGAGAAUGUCCAAAACAUG (SEQ ID NO: 1) |
| Circular RNA Circ-ACE2-open reading frame sequence | AUGUCAAGCUCUUCCUGGCUCCUUCUCAGCCUUGUUGCUG UAACUGCUGCUCAGUCCACCAUUGAGGAACAGGCCAAGAC AUUUUUGGACAAGUUUAACCACGAAGCCGAAGACCUGUUC UAUCAAAGUUCACUUGCUUCUUGGAAUUAUAACACCAAUA UUACUGAAGAGAAUGUCCAAAACAUGCGCCCAACCCAAGU UCAAAGGCUGAUAAGAGAGAAAAUCUCAUGA (SEQ ID NO: 2) |
| CircACE2-76aa polypeptide sequence | MSSSSWLLLSLVAVTAAQSTIEEQAKTFLDKFNHEAEDLFYQSSL ASWNYNTNITEENVQNMRPTQVQRLIREKIS (SEQ ID NO: 3) |
| CircACE2-76aa-FC amino acid sequence | MSSSSWLLLSLVAVTAAQSTIEEQAKTFLDKFNHEAEDLFYQSSL ASWNYNTNITEENVQNMRPTQVQRLIREKISLVPRGSGGGGDPE PKSCDKTHTCPPCPAPELLGGPSVFLFPPKPKDTLMISRTPEVTCV VVDVSHEDPEVKFNWYVDGVEVHNAKTKPREEQYNSTYRVVS VLTVLHQDWLNGKEYKCKVSNKALPAPIEKTISKAKGQPREPQV YTLPPSRDELTKNQVSLTCLVKGFYPSDIAVEWESNGQPENNYKT TPPVLDSDGSFFLYSKLTVDKSRWQQGNVFSCSVMHEALHNHYT QKSLSLSPGK (SEQ ID NO: 4) |
| CircACE2-76aa-FC fusion expression cassette nucleic acid sequence | ATGTCAAGCTCTTCCTGGCTCCTTCTCAGCCTTGTTGCTGTA ACTGCTGCTCAGTCCACCATTGAGGAACAGGCCAAGACATTT TTGGACAAGTTTAACCACGAAGCCGAAGACCTGTTCTA TCAAAGTTCACTTGCTTCTTGGAATTATAACACCAATATTAC TGAAGAGAATGTCCAAAACATGCGCCCAACCCAAGTTCAAA GGCTGATAAGAGAGAAAATCTCACTGGTGCCCAGAGGCT CCGGCGGCGGCGGCGATCCTGAGCCCAAATCTTGTGACAA AACTCACACATGCCCACCGTGCCCAGCACCTGAACTCCTGG GGGGACCGTCAGTCTTCCTCTTCCCCCCAAAACCCAAGG ACACCCTCATGATCTCCCGGACCCCTGAGGTCACATGCGTG GTGGTGGACGTGAGCCACGAAGACCCTGAGGTCAAGTTCA ACTGGTACGTGGACGGCGTGGAGGTGCATAATGCCAAGA CAAAGCCGCGGGAGGAGCAGTACAACAGCACGTACCGTGT GGTCAGCGTCCTCACCGTCCTGCACCAGGACTGGCTGAAT |
| | GGCAAGGAGTACAAGTGCAAGGTCTCCAACAAAGCCCTCC CAGCCCCCATCGAGAAAACCATCTCCAAAGCCAAAGGGCA GCCCCGAGAACCACAGGTGTACACCCTGCCCCCATCCCGG GATGAGCTGACCAAGAACCAGGTCAGCCTGACCTGCCTGG TCAAAGGCTTCTATCCCAGCGACATCGCCGTGGAGTGGGA GAGCAATGGGCAGCCGGAGAACAACTACAAGACCACGCCT CCCGTGCTGGACTCCGACGGCTCCTTCTTCCTCTACAGCAA GCTCACCGTGGACAAGAGCAGGTGGCAGCAGGGGAACGTC TTCTCATGCTCCGTGATGCATGAGGCTCTGCACAACCACTAC ACGCAGAAGAGCCTCTCCCTGTCTCCGGGTAAATGA (SEQ ID NO: 5) |
| Chemically synthesized CircACE2-76aa amino acid sequence | QSTIEEQAKTFLDKFNHEAEDLFYQSSLASWNYNTNITEENVQN MRPTQVQRLIREKIS (SEQ ID NO: 6) |
| CircACE2-76aa-mcherry circular RNA fusion expression cassette sequence | AGTGCTGAGATTACAGGCGTGAGCCACCACCCCCGGCCCACTT TTTGTAAAGGTACGTACTAATGACTTTTTTTTTATACTTCAGCG CCCAACCCAAGTTCAAAGGCTGATAAGAGAGAAAATCTCAAA GGGCGAGGAGGATAACATGGCCATCATCAAGGAGTTCATGCGC TTCAAGGTGCACATGGAGGGCTCCGTGAACGGCCACGAGTTC GAGATCGAGGGCGAGGGCGAGGGCCGCCCCTACGAGGGCAC CCAGACCGCCAAGCTGAAGGTGACCAAGGGTGGCCCCCTGCC CTTCGCCTGGGACATCCTGTCCCCTCAGTTCATGTACGGCTCCA AGGCCTACGTGAAGCACCCCGCCGACATCCCCGACTACTTGAA GCTGTCCTTCCCCGAGGGCTTCAAGTGGGAGCGCGTGATGAA CTTCGAGGACGGCGGCGTGGTGACCGTGACCCAGGACTCCTC CCTGCAGGACGGCGAGTTCATCTACAAGGTGAAGCTGCGCGG CACCAACTTCCCCTCCGACGGCCCCGTAATGCAGAAGAAGACT ATGGGCTGGGAGGCCTCCTCCGAGCGGATGTACCCCGAGGAC GGCGCCCTGAAGGGCGAGATCAAGCAGAGGCTGAAGCTGAA GGACGGCGGCCACTACGACGCTGAGGTCAAGACCACCTACAA |
| | GGCCAAGAAGCCCGTGCAGCTGCCCGGCGCCTACAACGTCAA CATCAAGTTGGACATCACCTCCCACAACGAGGACTACACCATC GTGGAACAGTACGAACGCGCCGAGGGCCGCCACTCCACCGGC GGCATGGACGAGCTGTACAAGTGAGGAGGTTTTAGTCTAGGG AAAGTCATTCAGTGGATGTGATCTTGGCTCACAGGGGACGATG TCAAGCTCTTCCTGGCTCCTTCTCAGCCTTGTTGCTGTAACTGC TGCTCAGTCCACCATTGAGGAACAGGCCAAGACATTTTTGGAC AAGTTTAACCACGAAGCCGAAGACCTGTTCTATCAAAGTTCAC TTGCTTCTTGGAATTATAACACCAATATTACTGAAGAGAATGTC CAAAACATGGTAAGAAGCAAGGAAAAGAATTAGGCTCGGCAC GGTAGCTCACACCTGTAATCCCAGCA (SEQ ID NO: 7) |

| | |
|---|---|
| Note: the underlined sequence is a circular RNA-mediated sequence | |

In the description of this specification, reference to the term "an embodiment", "some embodiments", "an example", "a specific example", or "some examples" or the like means that a specific feature, structure, material, or characteristic described in combination with the embodiment(s) or example(s) are included in at least one embodiment or example of the present disclosure. In this specification, illustrative expressions of these terms do not necessarily refer to the same embodiment or example. Moreover, the specific feature, structure, material, or characteristic described may be combined in any suitable manner in any one or more embodiments or examples. In addition, without mutual contradiction, those skilled in the art may incorporate and combine different embodiments or examples and features of the different embodiments or examples described in this specification.

Although the embodiments of the present disclosure have been illustrated and described above, it should be understood that the above embodiments are illustrative and cannot be construed as limitations on the present disclosure. Various changes, modifications, replacements, and variants may be made to the above embodiments by those skilled in the art without departing from the scope of the present disclosure.

## Claims

1. An isolated circular RNA molecule, wherein the circular RNA molecule comprises at least one of the following sequences:
1) an RNA sequence as set forth in SEQ ID NO: 1;
2) an RNA sequence having at least 70% identity with 1), preferably an RNA sequence having at least 80% identity with 1), preferably an RNA sequence having at least 85% identity with 1), preferably an RNA sequence having at least 90% identity with 1), preferably an RNA sequence having at least 95% identity with 1), and more preferably, an RNA sequence having at least 99% identity with 1).

2. The circular RNA molecule according to claim 1, wherein the circular RNA molecule is formed by a separate cyclization of a second exon of an mRNA sequence encoding angiotensin-converting enzyme 2;
optionally, the cyclization occurs in placental tissues.

3. The circular RNA molecule according to claim 1 or claim 2, wherein the circular RNA molecule is formed by linking a starting nucleotide and a terminal nucleotide of the nucleotide sequence as set forth in 1) or 2), the starting nucleotide being set as a first nucleotide of the circular RNA molecule.

4. The circular RNA molecule according to claim 3, wherein a coding region of the circular RNA molecule comprises nucleotides at position 104 to position 45 of the circular RNA molecule;
optionally, a nucleic acid sequence of the coding region of the circular RNA comprises at least one of the following sequences:
1) an RNA sequence as set forth in SEQ ID NO: 2;
2) an RNA sequence having at least 70% identity with 1), preferably an RNA sequence having at least 80% identity with 1), preferably an RNA sequence having at least 85% identity with 1), preferably an RNA sequence having at least 90% identity with 1), preferably an RNA sequence having at least 95% identity with 1), and more preferably, an RNA sequence having at least 99% identity with 1).

5. A polypeptide, wherein an amino acid sequence of the polypeptide comprises at least one of the following sequences:
1) an amino acid sequence as set forth in SEQ ID NO: 3;
2) an amino acid sequence having at least 70% identity with 1), preferably an amino acid sequence having at least 80% identity with 1), preferably an amino acid sequence having at least 85% identity with 1), preferably an amino acid sequence having at least 90% identity with 1), preferably an amino acid sequence having at least 95% identity with 1), and more preferably, an amino acid sequence having at least 99% identity with 1).

6. The polypeptide according to claim 5, wherein the polypeptide comprises a signal peptide sequence;
optionally, an amino acid sequence of the signal peptide is MS S S SWLLLSLVAVTAA.

7. The polypeptide according to claim 5 or claim 6, wherein the peptide is present in placental tissues.

8. A fusion protein, comprising the polypeptide according to any one of claims 5 to 7 and an Fc, wherein a C-terminal of the polypeptide is linked to an N-terminal of the Fc.

9. The fusion protein according to claim 8, wherein the fusion protein comprises at least one of the following sequences:
1) an amino acid sequence as set forth in SEQ ID NO: 4;
2) an amino acid sequence having at least 70% identity with 1), preferably an amino acid sequence having at least 80% identity with 1), preferably an amino acid sequence having at least 85% identity with 1), preferably an amino acid sequence having at least 90% identity with 1), preferably an amino acid sequence having at least 95% identity with 1), and more preferably, an amino acid sequence having at least 99% identity with 1).

10. An isolated nucleic acid encoding the polypeptide according to any one of claims 5 to 7 or the fusion protein according to claim 8 or claim 9.

11. The nucleic acid according to claim 10, wherein the nucleic acid encoding the fusion protein according to claim 8 or claim 9 comprises at least one of the following sequences:
1) a nucleotide sequence as set forth in SEQ ID NO: 5;
2) a nucleotide sequence having at least 70% identity with 1), preferably a nucleotide sequence having at least 80% identity with 1), preferably a nucleotide sequence having at least 85% identity with 1), preferably a nucleotide sequence having at least 90% identity with 1), preferably a nucleotide sequence having at least 95% identity with 1), and more preferably, a nucleotide sequence having at least 99% identity with 1).

12. Use of the RNA molecule according to any one of claims 1 to 4, the polypeptide according to any one of claims 5 to 7, the fusion protein according to claim 8 or claim 9, the isolated nucleic acid according to claim 10 or claim 11 in the preparation of a medicament or a kit for inhibiting the infection of cells by COVID-19.

13. A method for inhibiting the infection of cells by COVID-19, comprising:
contacting the COVID-19 or cells to be infected with the RNA molecule according to any one of claims 1 to 4, the polypeptide according to any one of claims 5 to 7, the fusion protein according to claim 8 or claim 9, or the isolated nucleic acid according to claim 10 or claim 11.

14. The method according to claim 13, wherein the contacting is carried out by the following steps:
1) performing a first mixing treatment of the COVID-19 with the RNA molecule according to any one of claims 1 to 4, the polypeptide according to any one of claims 5 to 7, the fusion protein according to claim 8 or claim 9, or the isolated nucleic acid according to claim 10 or claim 11;
2) performing a second mixing treatment of the mixture obtained in step 1) with the cells to be infected to inhibit the infection of the cells by the COVID-19.

15. The method according to claim 13, wherein a final concentration of the polypeptide or the fusion protein in a mixed system is 10 µg/mL or 160 µg/mL.

16. A method for treating or preventing novel coronavirus pneumonia, comprising administering to a patient the pharmaceutically acceptable RNA molecule according to any one of claims 1 to 4, the polypeptide according to any one of claims 5 to 7, the fusion protein according to claim 8 or claim 9, or the isolated nucleic acid according to claim 10 or claim 11.

17. Use of the RNA molecule according to any one of claims 1 to 4, the polypeptide according to any one of claims 5 to 7, the fusion protein according to claim 8 or claim 9, or the isolated nucleic acid according to claim 10 or claim 11 in the treatment or prevention of novel coronavirus pneumonia.
